(19) Europäisches Patentamt / European Patent Office / Office européen des brevets

(11) **EP 3 868 334 A1**

(12) **EUROPEAN PATENT APPLICATION**
published in accordance with Art. 153(4) EPC

(43) Date of publication:
**25.08.2021 Bulletin 2021/34**

(51) Int Cl.:
**A61C 19/04** (2006.01)    **A61B 10/00** (2006.01)
**A61C 19/06** (2006.01)

(21) Application number: **19874232.2**

(22) Date of filing: **09.10.2019**

(86) International application number:
**PCT/JP2019/039804**

(87) International publication number:
**WO 2020/080219 (23.04.2020 Gazette 2020/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.10.2018 JP 2018196773**

(71) Applicant: Osaka University
Suita-shi, Osaka 565-0871 (JP)

(72) Inventors:
• **HAZAMA, Hisanao**
  **Suita-shi, Osaka 565-0871 (JP)**
• **AWAZU, Kunio**
  **Suita-shi, Osaka 565-0871 (JP)**
• **YOSHIKAWA, Kazushi**
  **Hirakata-shi, Osaka 573-1121 (JP)**

(74) Representative: **Müller-Boré & Partner**
**Patentanwälte PartG mbB**
**Friedenheimer Brücke 21**
**80639 München (DE)**

(54) **OPTICAL DENTAL CARIES DIAGNOSTIC INSTRUMENT, OPTICAL DENTAL CARIES DIAGNOSTIC METHOD, AND OPTICAL DENTAL CARIES DIAGNOSTIC SYSTEM**

(57)    Disclosed is an optical dental caries diagnostic device and an optical diagnostic device for periodontal diseases that can quantitatively diagnose progression of dental caries or periodontal diseases, and an optical diagnostic method including the same. Also disclosed is a diagnostic system including such diagnostic devices.

The optical dental caries diagnostic device (1) has a probe (30) having optical transparency and a taper shape with an end portion to be in contact with a tooth, a light source (20) emitting light to the probe (30), and a light receiving element (40) receiving light from the end portion of the probe (30).

[Fig. 2A]

EP 3 868 334 A1

[Fig. 2B]

**Description**

TECHNICAL FIELD

The present invention relates to an optical dental caries diagnostic device, which may be handheld, an optical diagnostic method for dental caries, and a diagnostic system including the optical dental caries diagnostic device and the optical diagnostic method for dental caries, and an optical diagnostic device for periodontal diseases.

BACKGROUND ART

Prevention and appropriate treatment of dental caries and periodontal diseases are societal demands to maintain our health of teeth and gums, and eventually general health. Especially for elderly people, since it is known that there is a strong correlation between the number of remaining teeth and incidence of dementia, maintenance of dental health is said to be effective to prevent dementia. If the incidence of dementia can be decreased by preventive dental health care, significant reduction in expense for nursing care can be expected.

Dental hard tissue (enamel and dentin) is demineralized and decayed when it contracts caries. While minor symptoms such as white spots in the enamel can be treated with a wait-and-see approach, teeth developing advanced dental caries need to be drilled. In conventional diagnosis and treatments of dental caries, it is the fact that distinguishing teeth developing dental caries from healthy teeth or selection of treatment depends on feelings of dentists. For example, dentists conventionally distinguish teeth developing dental caries from healthy teeth with a dental probe or staining fluid depending on their feelings, and have not got a quantitative guideline. When they drill diseased teeth, they also depend on their feelings, and sometimes drill a tooth that is essentially healthy and needs not to be drilled. Therefore, a technology with which dentists can diagnose how much dental caries progresses without their feelings has been required.

In addition, while fluorine and the like is applied to teeth to reduce dental caries progression, it is important to know the stage of dental caries to appropriately apply fluorine and the like based on quantitative evaluation of the progression of dental caries.

Among elderly people, the incidence rate of root caries, which develops at the root of a tooth, is more than 50 %, however, a technology that can quantitatively evaluate the stage of root caries has not been established. Especially, it is difficult to diagnose an intermediate case between the case where a carious tooth should be drilled and the case requiring only preventive care. From this point of view, improvement of a technology with which dentists can diagnose progression of dental caries is required.

"A dental caries diagnosis devise" is disclosed in patent document 1 as one of the technologies for dental caries diagnosis. The devise determines whether softened dentin is present or not by pressing a probe on a lesion and pulling it up to learn whether resistance (tug back) is generated or not. However, while the device can determine the presence or absence of the softened dentin, it cannot measure a degree of softening to quantify the progression of softening.

"A dental caries diagnosis devise" disclose in patent document 2 diagnoses dental caries by radiating light to adjacent surface of teeth and measuring intensity of generated fluorescence. However, by this devise, quantitative evaluation of the progression of dental caries is also difficult. Furthermore, the opportunity of using this devise is restricted because it is not supposed to be used for teeth surface other than the adjacent surface, and it requires filler between the teeth and the probe, which makes the diagnosis complicated.

"A probe for measuring hardness and a method using the same" disclosed in patent document 3 tried to quantitatively measure hardness. The patent document 3 discloses a method in which paint is applied to a surface of a probe having a cone shape, and the probe is pressed to an object to be measured with a predetermined force, which leads to a loss of paint at a part of the probe that has sunk in the object. Using this mechanism, a length of the part that shows the loss of paint is measured with a microscope to determine hardness of the object. However, the method disclosed in the patent document 3, which includes the step of microscope observation on top of the step of applying paint to the surface of the probe having a cone shape, needs more than one steps and some apparatuses, and measurement procedure of which is cumbersome.

RELATED ART DOCUMENTS

PATENT DOCUMENTS

Patent Document 1: JP-A-2008-29412

Patent Document 2: JP-A-2010-252911

Patent Document 3: JP-A-2011-112629

SUMMARY OF THE INVENTION

PROBLEMS TO BE SOLVED BY THE INVENTION

Based on the above circumstances, the purpose of the present invention is to provide an optical dental caries diagnostic device and an optical diagnostic device for periodontal diseases that can quantitatively diagnose progression of dental caries or periodontal diseases, and an optical diagnostic method including the same. For example, the device may be a small apparatus that can be handheld. Furthermore, the purpose of the present invention is to provide a diagnostic system including such diagnostic devices, not only to contribute to prevention or diagnosis of dental caries and periodontal diseases, but also to enable minimally invasive dental therapy.

MEANS FOR SOLVING THE PROBLEMS

The present inventors repeated intensive studies in order to solve the above-described problems. As a result, the inventors completed the present invention by finding that hardness of teeth and gum can be quantitatively measured by emitting light to a probe being in contact with a tooth or gum, and receiving the light reflected by the probe.
An optical dental caries diagnostic device, an optical device for periodontal diseases, an optical diagnostic method for dental caries, and a diagnostic system including the device are as follows.

[1] An optical dental caries diagnostic device, comprising:

a probe having optical transparency and a taper shape with an end portion to be in contact with a tooth,
a light source emitting light to the probe, and
a light receiving element having a light receiving surface and receiving light from the end portion of the probe.

[2] The optical dental caries diagnostic device according to [1], further comprising a lens disposed at a position where the end portion of the probe and the light receiving surface of the light receiving element establish an imaging relationship.
[3] The optical dental caries diagnostic device according to [1] or [2], wherein the light receiving element includes unit cells arrayed in two dimensions.
[4] The optical dental caries diagnostic device according to any one of [1] to [3], further comprising a beam splitter disposed at a position that is in an optical path between the probe and the light source and is in an optical path between the probe and the light receiving element.
[5] The optical dental caries diagnostic device according to any one of [1] to [4], further comprising a pressure sensor, wherein the light receiving element takes an image when the pressure sensor shows a predetermined pressure generated by the probe being in contact with a tooth.
[6] The optical dental caries diagnostic device according to any one of [1] to [5], wherein a differential intensity image is obtained by subtracting a received light intensity by the light receiving element while the end portion of the probe is in contact with a tooth from a received light intensity by the light receiving element while the end portion of the probe is in contact with nothing.
[7] The optical dental caries diagnostic device according to [6], wherein the differential intensity image is binarized in accordance with a predetermined threshold to obtain a binarized differential intensity image.
[8] The optical dental caries diagnostic device according to [7], wherein the device generates an error signal when a ratio (a length of a major axis/a length of a minor axis) of a profile of the binarized differential intensity image is greater than a predetermined value.
[9] The optical dental caries diagnostic device according to [7] or [8], obtaining an area of a region of the binarized differential intensity image over the threshold.
[10] The optical dental caries diagnostic device according to any one of [1] to [9], further comprising a housing, wherein the probe is attachable to and detachable from the housing.
[11] An optical diagnostic method for dental dental caries, comprising:

preparing the optical dental caries diagnostic device according to any one of [1] to [10];
removing water from the end portion of the probe;
receiving light that is emitted by the light source and reflected from the end portion of the probe to take an image;
bringing the end portion of the probe into contact with a tooth; and
receiving light that is emitted by the light source and reflected from the end portion of the probe to take an image while the end portion of the probe is in contact with the tooth.

[12] A diagnostic system for dental caries including the optical dental caries diagnostic device according to any one of [1] to [10], comprising:

an information terminal device connected by wire or wirelessly to the optical dental caries diagnostic device; and optical dental caries diagnostic information (measured with the optical dental caries diagnostic device according to any one of [1] to [10]) stored in the information terminal device.

[13] The diagnostic system for dental caries according to [12], wherein the information terminal device has dental caries diagnostic information measured with a device other than the optical dental caries diagnostic device according to any one of [1] to [10].

[14] An optical diagnostic device for periodontal diseases, comprising:

a probe having optical transparency and a taper shape with an end portion to be in contact with a gum; a light source emitting light to the probe, and a light receiving element receiving light from the end portion of the probe.

EFFECTS OF THE INVENTION

Teeth and gum developing caries or periodontal diseases become soft losing their intrinsic hardness. Focusing on this mechanism, the optical dental caries diagnostic device, the optical diagnostic method for dental caries, the optical diagnostic device for periodontal diseases, and the diagnostic system including the devices of the present invention can quantitatively measure the hardness of teeth or gum by quantitatively measuring reflected light from the probe being in contact with the teeth to diagnose progression of dental caries. Thereby, dental caries and periodontal diseases can be discriminated, and progression thereof can be quantitatively diagnosed not depending on dentists' experience. In addition, not only measuring or diagnosing, the diagnostic system of the present invention also can accumulate information by managing the mensural or diagnostic outcome with applications used in personal computers, tablets, smart phones, and the like. Utilizing the information to support dentists not only allows minimally invasive dental therapy, but also is useful for the prevention of dental caries and periodontal diseases, which can promises substantial reduction in medical costs.

BRIEF DESCRIPTION OF THE DRAWINGS

Fig. 1A is a lateral view of a probe in accordance with one embodiment of the present invention.
Fig. 1B is a plan view of the probe shown in Fig. 1A.
Fig. 1C is a lateral view of a probe in accordance with another embodiment of the present invention.
Fig. 2A is a cross-sectional view of an optical dental caries diagnostic device in accordance with one embodiment of the present invention.
Fig. 2B is a cross-sectional view at another cross section of the optical dental caries diagnostic device shown in Fig. 2A.
Fig. 3 is a cross-sectional view on line III-III of the optical dental caries diagnostic device shown in Fig. 2B.
Fig. 4 is an image of reflected light from an end portion of a probe taken while the end portion of the probe is in contact with nothing (non-contact).
Fig. 5A is an image of reflected light from an end portion of a probe taken while the end portion of the probe is in contact with a tooth having healthy dentin.
Fig. 5B is an image of reflected light from an end portion of a probe taken while the end portion of the probe is in contact with a tooth having decalcified dentin (dental caries model).
Fig. 6 is a lateral view when light is emitted to the probe shown in Fig. 1A.
Fig. 7 is a graph showing a relationship between time and contact pressure.
Fig. 8A is an image of reflected light from an end portion of a probe taken while the end portion of the probe is non-contact.
Fig. 8B is an image of reflected light from the end portion of the probe taken while the end portion of the probe is in contact with a tooth.
Fig. 8C is a differential intensity image obtained by subtracting the image intensity of Fig. 8B from the image intensity of Fig. 8A.
Fig. 8D is a binarized differential intensity image obtained by binarizing the differential intensity image of Fig. 8C.

Fig. 9A is a schematic view when an end portion of a probe is perpendicularly in contact with a tooth.
Fig. 9B is a schematic view when an end portion of a probe is not perpendicularly in contact with a tooth.
Fig. 10 is a cross-sectional view of a simulator of an optical dental caries diagnostic device in accordance with an embodiment of the present invention.
Fig. 11 is a graph showing a relationship between Knoop hardness number and an area of a binarized differential intensity image.

MODE FOR CARRYING OUT THE INVENTION

Hereinafter, the present invention will be specifically described with reference to embodiments. The present invention, however, is not limited by the following embodiments, and can be put into practice with appropriate changes within a range meeting the gist of the above and the below, all of which are included in the technical scope of the present invention. In the drawings, hatching and a reference sign for a member may be omitted for convenience, and in such a case, the description and other drawings should be referred to. In addition, sizes of various members in the drawings may differ from the actual sizes thereof, since priority is given to understanding the features of the present invention.

An optical dental caries diagnostic device of the present invention has a light source, a probe having an end portion that is to be in contact with a tooth, and a light receiving element. The probe is composed of a material having optical transparency, and the end portion of the probe has a taper shape. When light emitted from the light source enters the probe, the incident light is reflected by the end portion of the probe. At the time, if the end portion of the probe is in the air without being in contact with anything, the incident light travels from a medium having a higher refractive index (a material composed of the probe) to a medium having a lower refractive index (air), and is reflected by the end portion of the probe. In this case, the reflection is total reflection depending on the incidence angle, which is received by the light receiving element. On the other hand, when the end portion of the probe is in contact with a tooth, total reflection is unlikely to occur, and as a result, the light receiving element receives weaker reflected light. Measuring the deference of intensity between the total reflection and the weaker reflected light enables to obtain information about the tooth with which the probe is in contact.

Generally, in a case where light travels perpendicularly from a material B having a refractive index $n_B$ to a material A having a refractive index $n_A$, a surface reflectivity Rref at a surface of the material A is given by the following formula (1).

$$R_{ref} = [(n_B - n_A)/(n_B + n_A)]^2 \qquad (1)$$

Accordingly, given that a reflective index of the probe is defined as $n_p$ and a reflective index of a tooth is defined as $n_t$, since the reflective index of the air is 1.0, a reflection ratio when light travels from the probe to the air is given by the following formula (2), and a reflection ratio when light travels from the probe to the tooth is given by the following formula (3).

$$R_{ref(probe\ to\ air)} = [(n_p - 1.0)/(n_p + 1.0)]^2 \qquad (2)$$

$$R_{ref(probe\ to\ tooth)} = [(n_p - n_t)/(n_p + n_t)]^2 \qquad (3)$$

Since the probe is solid and has a reflective index peculiar to solid materials, $R_{ref(probe\ to\ air)}$ is always greater than $R_{ref(Probe\ to\ tooth)}$ ($R_{ref(probe\ to\ air)} > R_{ref(probe\ to\ tooth)}$). While the above formulae (1) to (3) are premised on the case where light enter into a surface perpendicularly (incidence angle = 0), in a case where the incidence angle is not 0 and does not allow total reflection, $R_{ref(probe\ to\ air)}$ is always greater than $R_{ref(probe\ to\ tooth)}$ (The case of total reflection will be described later.). Accordingly, the larger an area of the end portion of the probe that is in contact with a tooth is, the lower the reflected light intensity is detected by the light receiving element, because a part of the probe that is in contact with a tooth has weaker reflected light intensity, and a part of the probe that is in contact with nothing has stronger reflected light intensity. Note that the softer a tooth is, the deeper the end portion of the probe enters into the tooth. Therefore, the softer a tooth is, the larger the area of the end portion of the probe that is in contact with the tooth is; the harder a tooth is, the smaller the area of the end portion of the probe that is in contact with the tooth. Accordingly, the softer a tooth is, the larger the area of the end portion of the probe that is in contact with the tooth is, and therefore the weaker the reflected light intensity is; the harder a tooth is, the smaller the area of the end portion of the probe that is in contact with the tooth is, and therefore, the strong the reflected light intensity is. In this manner, measuring the reflected light intensity detected by the light receiving element enables to quantitatively measure hardness of teeth.

As described above, the optical dental caries diagnostic device of the present invention enables quantitative measurement of hardness of teeth to diagnose dental caries by measuring reflected light from the end portion of the probe that is in contact with a tooth. According to the device, the hardness of teeth can be quantitatively measured without conventional complicated procedure that needs filler, paint, a microscope, and the like. In addition, since the device can be configured to be portable, the device can measure hardness of teeth to diagnose dental caries anywhere.

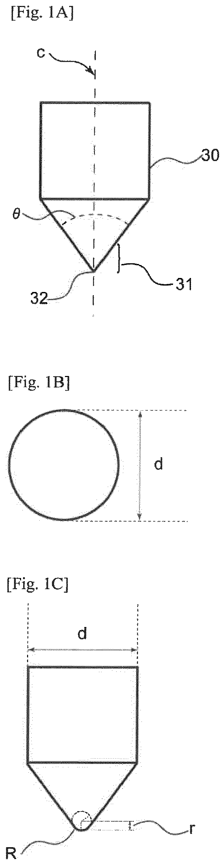

Hereinafter, an optical dental caries diagnostic device 1 according to an embodiment of the present invention will be specifically described in reference to Fig. 1 to Fig. 6.

An optical dental caries diagnostic device 1 has a light source 20, a probe 30 that is to be in contact with a tooth, and a light receiving element 40.

The light source 20 is not particularly limited, and may include semiconductor laser and light-emitting, which are suitable because light having specific wavelength can be obtained, and therefore unwanted light can be easily eliminated. Of these, the semiconductor laser is preferable since it has directionality.

The probe 30 is composed of a material having optical transparency, and an end portion 31 of the probe 30 is made to have a taper shape by reducing its diameter. A favorable shape of the end portion 31 of the probe 30 is an approximately cone shape including a polygonal pyramid as well as a cylindrical cone shown in Fig. 1 as an example. Of these, a cylindrical cone and a quadrangular pyramid are preferable. The end portion 31 has a tip 32. In the case the end portion 31 has a cylindrical cone shape or a polygonal pyramid shape, the tip 32 corresponds to its apex. The probe 30 has a surface that is located opposite to the tip 32 (hereinafter referred to as "base"), and the shape of the surface may be a circle or a polygonal shape, or may be another different shape.

Fig. 1 shows an example of the probe 30, and Fig. 1A shows a lateral view of the probe 30 having a circular cylinder and a cylindrical cone having reduced diameter. Fig. 1B is a plan view where Fig. 1A is viewed from directly above. The probe 30 has a central axis c connecting a center of a circle (base) having a diameter d and an apex of the cylindrical cone, and the tip 31 of the cylindrical cone has an angle θ.

An area of the surface perpendicular to the center axis c of the probe 30 is not particularly limited as long as the probe 30 can be inserted into the oral cavity, and the diameter or the major diameter of the surface is preferably 3 mm or less, and more preferably 2 mm or less. The angle θ of the end portion 31 having reduced diameter is, for example, an angle between generating lines in the case where the shape of the end portion 31 is a cylindrical cone as shown in Fig. 1, or an angle between diagonal pair of surfaces in the case where the shape of the end portion 31 is a quadrangular pyramid, which hereinafter may be referred to as simply θ.

As shown in Fig. 1C, the tip 32 of the end portion 31 of the probe 30 is not necessarily keen-edged, but may have a roundness to some extent (R portion), and the shape of the tip may be appropriately selected according to the patient's situation including hardness or shape of teeth, progression of dental caries, and the patient's age. The tip 32 having a roundness to some extent has the advantage of being unlikely to be damaged and unlikely to cause pain to the patient. On the other hand, if the tip 32 has excessive roundness, it may become harder for the tip 32 to be in contact with a tooth, or the excessive roundness may have a bad effect on detecting reflected light. Accordingly, while a curvature radius r of the R portion can be appropriately selected as long as it meets the above requirement, the curvature radius r is preferably 5 $\mu$m or more, more preferably 10 $\mu$m or more, even more preferably 30 $\mu$m or more, and preferably 500 $\mu$m or less, more preferably 200 $\mu$m or less, even more preferably 150 $\mu$m or less.

A material constituting the probe 30 is not particularly limited as long as it has optical transparency, and appropriately selected from materials such as glass, mineral, and resin. Specifically, the material is exemplified by glass including glass and organic glass; natural ore including sapphire and ruby; artificial mineral; resin materials including acrylic resin, polycarbonate resin, polyester resin, and polyvinyl chloride resin. Among these, a material can be selected to make the probe 30 so that the reflective index and the hardness of the material are optimum for the condition of teeth.

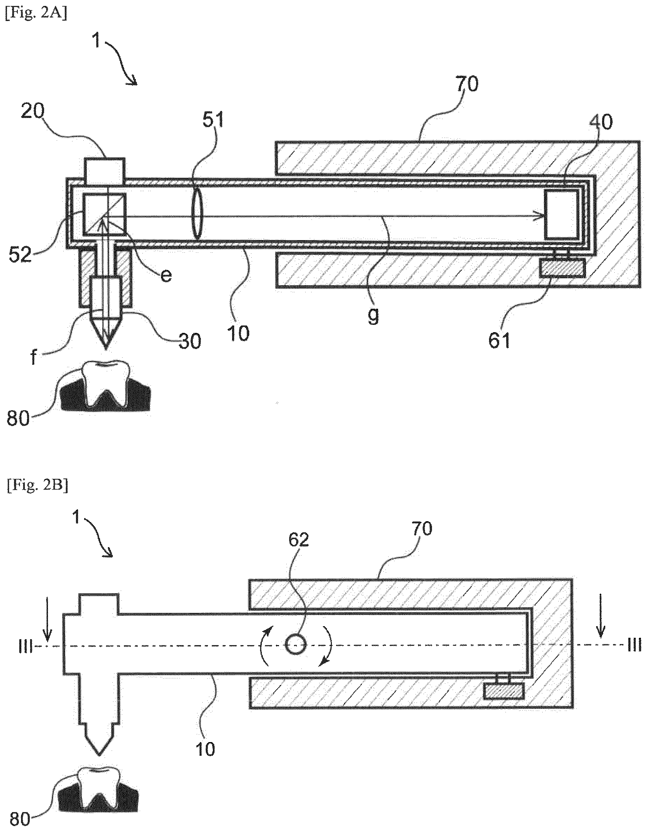

As shown in Fig. 2A, the optical dental caries diagnostic device 1 of the present invention preferably further has a lens 51 at a position where the end portion 31 of the probe 30 and the light receiving surface of the light receiving element 40 establish an imaging relationship. Imaging reflected light from the end portion 31 of the probe 30 by the lens 51 enables the light receiving element 40 to detect contrast between a region showing weaker reflected light and a region showing stronger reflected light. Furthermore, it has the advantage of reduced variation in calibration relative to a method in which the intensity of reflected light is simply measured without a lens.

Alternatively, the lens 51 may be disposed at a position where a position predetermined distance deviated from the tip 32 of the end portion 31 of the probe 30 to the base of the probe 30 and the light receiving surface of the light receiving element 40 establish an imaging relationship. Such a configuration enables the light receiving element 40 to detect contrast between a region showing weaker reflected light and a region showing stronger reflected light more accurately. The above predetermined distance is more than 0, and preferably three times the curvature radius r of the R portion or less, more preferably twice the curvature radius r of the R portion or less.

The light receiving element 40 may include unit cells arrayed in two dimensions. The unit cells arraying in two dimensions may be solid-state image sensing devices (image sensors) including a CCD image sensor, and a CMOS image sensor. Of these, the CMOS image sensor is preferable for reasons of its reasonable supply price, and its portable design with little power consumption.

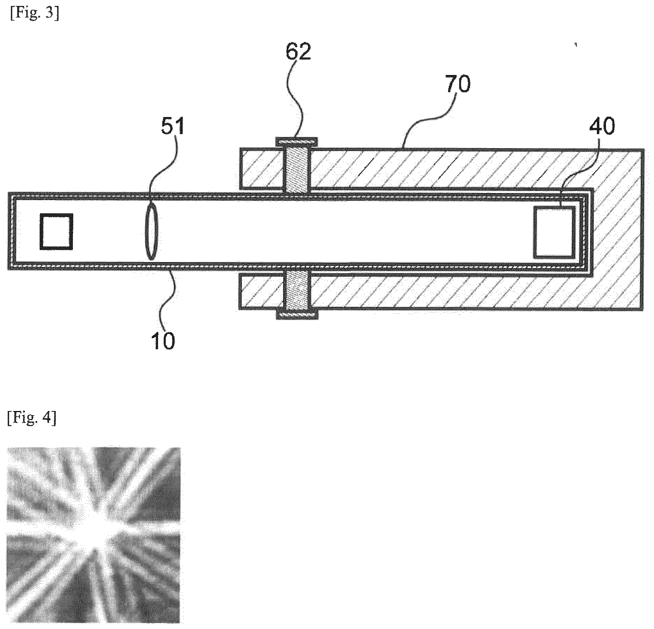

Fig. 4 shows an example of images of reflected light formed by the lens 51 taken by the light receiving element 40 while the end portion 31 of the probe 30 is in contact with nothing. The center part of the image shown in Fig. 4 appears to be white because strong reflected light from the end portion 31 of the probe 30 is measured to be taken as an image having high contrast.

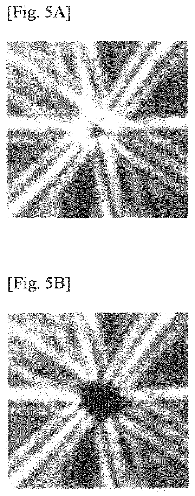

Fig. 5A shows an example of images of reflected light formed by the lens 51 taken by the light receiving element 40 while the end portion 31 of the probe 30 is in contact with a tooth having healthy dentin, and Fig. 5B shows an example of images of reflected light formed by the lens 51 taken by the light receiving element 40 while the end portion 31 of the probe 30 is in contact with a tooth having decalcified dentin (dental caries model). When the end portion 31 of the probe 30 is in contact with a tooth, reflected light from a region that is in contact with the tooth of the end portion 31 becomes weak, and as the result, an image having a black part in the center as shown in Fig. 5A, which corresponds to the region of weakened reflected light, is obtained. When the end portion 31 of the probe 30 is in contact with a tooth having decalcified dentin (dental caries model), an area of a region that is in contact with the tooth of the end portion 31 become large, and as the result, an image having a larger black part in the center as shown in Fig. 5B, which corresponds to the region of weakened reflected light, is obtained.

An area of the end portion 31 of the probe 30 that is in contact with a tooth depends on hardness of the tooth. Since an area of a region of weakened reflected light corresponds to the area of the end portion 31 of the probe 30 that is in contact with a tooth, measuring the area of the region of weakened reflected light can determine hardness of the tooth. For example, by comparing the area of weakened reflected light (black part in the center) shown in Fig. 5A to the area of weakened reflected light (black part in the center) shown in Fig. 5B, hardness of the tooth with which the image shown in Fig. 5A is obtained and hardness of the tooth with which the image shown in Fig. 5B is obtained can be quantitatively compared.

Since the end portion 31 of the probe 30 has the angle $\theta$, light that enters from a side of the base of the probe 30 reaches the end portion 31 at the incidence angle depending on the angle $\theta$, and is reflected. When the light travels from the end portion 31 of the probe 30 to the air (non-contact), the reflection is total reflection, and therefore, if the probe 30 has $\theta$ that does not lead to total reflection while being in contact with a tooth, contrast between a region of strong reflected light and a region of weak reflected light can be measured more clearly.

The total reflection is a phenomenon in which when light travels from a medium B having a greater refractive index to a medium A having a smaller refractive index (Given that the refractive index of the medium A is $n_A$ and the refractive index of the medium B is $n_B$, $n_B > n_A$ is satisfied.), the incident light does not penetrate the boundary surface, and all of the incident light is reflected. The phenomenon occurs under the condition that the incidence angle is greater than or equal to a critical angle, which is given by the following formula (4) in accordance with Snell's law.

$$\sin\theta_C = n_A/n_B \qquad (4)$$

Since solid substance generally has a refractive index greater than the air's refractive index of 1.0, a refractive index $n_p$ of the probe 30 satisfies $n_p > 1.0$. When the probe 30 is non-contact, since light travels from the end portion 31 of the probe 30 to the air, the total reflection occurs at the end portion 31 under the condition that the incidence angle is larger than $\theta_{ca}$, which is a critical angle when light travels from the probe 30 to the air, given by the following formula (5), which is given by substituting $n_A = 1.0$, and $n_B = n_p$ to the formula (4).

$$\sin \theta_{Ca} = 1.0 \text{ (refractive index of the air)}/n_p \text{ (refractive index of the probe 30)} \qquad (5)$$

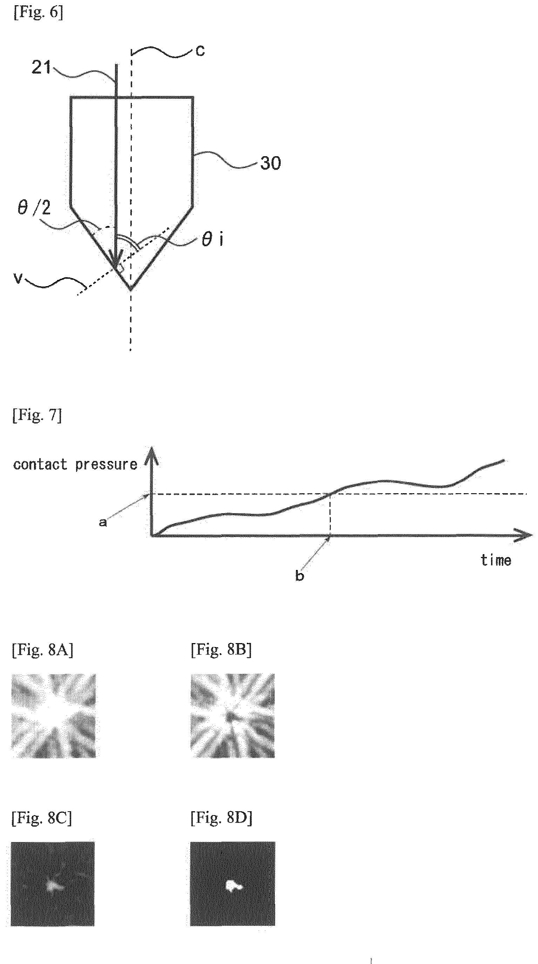

Given that the light from the light source 20 enters the probe 30 parallel to the central axis c of the probe 30 similarly to light 21 shown in Fig. 6, an angle between the light 21 and the generating line of the end portion 31 of the probe 30 is equal to $\theta/2$. Since an incidence angle $\theta_i$ of the light 21 is an angle between the light 21 and a perpendicular line v to the generating line, the incidence angle $\theta_i$ can be obtained by the following formula (6). When the light travels from the probe 30 to the air, the total reflection at the end portion 31 of the probe 30 that is non-contact occurs under the condition that the following formula (8) is satisfied, because the total reflection at the end portion 31 occurs in the case where $\theta_i$ is larger than $\theta_{ca}$ (as shown in the following formula (7)).

$$\theta_i = 90 - \theta/2 \qquad (6)$$

$$\theta_i > \theta_{Ca} \qquad (7)$$

$$\theta \leq 2(90 - \theta_{Ca}) \quad (8)$$

Next, when the end portion 31 of the probe 30 is in contact with a tooth, reflection from a region that is in contact with the tooth of the end portion 31 is different depending on whether $n_p$ is greater than $n_t$ or $n_p$ is not greater than $n_t$, given that the refractive index of the tooth is $n_t$.

[In the case of $n_p \leq n_t$]

In the case of $n_p \leq n_t$, the light travels from the end portion 31 of the probe 30 having a smaller refractive index to a tooth having a greater refractive index, and therefore, the reflection at the region that is in contact with the tooth of the end portion 31 is not the total reflection. As a result, when $\theta$ satisfies the above formula (8), while the reflection at a region that is non-contact is the total reflection, the reflection at a region that is in contact with the tooth is not the total reflection, which leads to a difference in contrast of the intensity of the received light by the light receiving element 40. Since the difference depends on whether the end portion 31 is in contact with the tooth or non-contact, obtaining an area of weakened received light intensity enables hardness of the tooth to be measured.

[In the case of $n_p > n_t$]

In the case of $n_p > n_t$, the light travels from the end portion 31 of the probe 30 having a greater refractive index to a tooth having a smaller refractive index, and therefore, the reflection at the region that is in contact with the tooth of the end portion 31 is also the total reflection when the light enters the probe 30 at an incidence angle that is greater than or equal to a critical angle $\theta_{Ct}$ of this case, which is a critical angle when the light travels from the probe 30 to a tooth ($\theta_i \geq \theta_{ct}$). On the other hand, the total reflection does not occur when the light enters the probe 30 at an incidence angle that is smaller than the critical angle $\theta_{Ct}(\theta_i < \theta_{Ct})$. Substituting $\theta_i = 90 - \theta/2$, which is the formula (6), into the formula $\theta_i \geq \theta_{Ct}$, when $\theta_{ct}$ satisfies the following formula (9), the total reflection does not occur at the region that is in contact with the tooth of the end portion 31, which can be observed in high contrast with a region that is in contact with nothing.

$$\theta > 2(90 - \theta_{Ct}) \quad (9)$$

In the case where the total reflection occurs at a region that is non-contact of the end portion 31 of the probe 30 and the total reflection does not occur at a region that is in contact with a tooth of the end portion 31 of the probe 30, the difference can be observed in high contrast. Therefore, $\theta$ preferably satisfies the following formula (10) on the basis of the above formulae (8) and (9). When $\theta$ is in this range, while the total reflection occurs at a region that is non-contact of the end portion 31 of the probe 30, the total reflection does not occur at a region that is in contact with a tooth, which leads to high contrast in light intensity received by the light receiving element 40. By obtaining an area of the region having weak received light intensity of an image taken by the light receiving element 40, hardness of a tooth can be measured.

$$2(90 - \theta_{Ct}) < \theta \leq 2(90 - \theta_{Ca}) \quad (10)$$

Since the critical angle $\theta_{Ct}$ at a region that is in contact with a tooth of the probe 30 is given by the following formula (11), and the refractive index of a tooth nt is greater than the refractive index of the air of 1.0, $\theta_{Ct}$ is always greater than $\theta_{ca}$. The larger the difference between $\theta_{Ct}$ and $\theta_{Ca}$ is, the wider the available range of $\theta$ is. Therefore, the larger difference is preferable because it leads to more choices of the probe 30.

$$\sin \theta_{Ct} = n_t \text{ (refractive index of a tooth)} / n_p \text{ (refractive index of the probe 30)} \quad (11)$$

In a case where light traveling from the light source 20 to the probe 30 includes a component that is not parallel to the central axis c of the probe 30, that is, the incidence angle is not constant, the probe 30 preferably has the refractive index $n_p$ such that the difference between $\theta_{Ct}$ and $\theta_{Ca}$ is larger. Such a configuration widens the range of angle in which the reflected light intensity at a region of non-contact is strong and the reflected light intensity at a region that is in contact with a tooth is weak, which is advantageous in obtaining high contrast.
Given that the refractive index of enamel of teeth is about 1.63 and the refractive index of dentin is about 1.55, in the case, for example, where a glass probe having a refractive index of 1.50 is used, since the refractive index of the probe is smaller than the refractive index of the tooth, $\theta$ is preferably less than or equal to 96° ($\theta \leq 96°$) on the basis of the formula (8) from the above viewpoint of the total reflection. Alternatively, in the case, for example, where a sapphire probe having a refractive index of 1.79 is used, since the refractive index of the probe is greater than the refractive index of the tooth, on the basis of the formula (10) from the above viewpoint of the total reflection, $\theta$ is preferably greater than 48° and less than or equal to 112° ($48° < \theta \leq 112°$) when enamel is measured, and $\theta$ is preferably greater than 60° and less than or equal to 112° ($60° < \theta \leq 112°$). As shown above, preferable range of $\theta$ can be calculated by substituting a refractive index of the probe that is to be used into the above formulae (8) to (10). Furthermore, taking into consideration various conditions such as patients' physical conditions and easiness of inserting the probe into the site that is to be measured, optimal $\theta$ can be determined.

In the above description, preferable $\theta$ for a probe having a cone shape has been described as an example, however, the similar explanation is available even when the end portion 31 of the probe 30 has another shape.

The optical dental caries diagnostic device 1 of the present invention may further has a beam splitter 52 disposed at a position that is in an optical path between the probe 30 and the light source 20 and is in an optical path between the probe 30 and the light receiving element 40. The beam splitter 52 enables an optical axis from the light source 20 and an optical axis to the light receiving element 40 to be different optical axes, and as a result, allows greater flexibility in the design of the optical dental caries diagnostic device 1. Consequently, the optical dental caries diagnostic device 1 can have a shape that is suitable for inserting the side of the probe 30 into the oral cavity while the other side of the device is held to control the device. In order to improve a S/N ratio of received signals, a reflectance of the beam splitter 52 is preferably, for example, 52 % or more, more preferably 55 % or more, and even more preferably 60 % or more.

The optical dental caries diagnostic device 1 of the present invention may further has a pressure sensor 61. When the pressure generated by the end portion 31 of the probe 30 being in contact with a tooth reaches a predetermined pressure, the light receiving element 40 takes an image in response to a signal from the pressure sensor 61. Such a configuration enables hardness measurement of a tooth under a constant pressure, which leads to quantitative measurement that does not depend on dentists' skill. Fig. 7 is a graph showing a relationship between time and contact pressure. In Fig. 7, "a" means the predetermined contact pressure, and "b" is the timing when an image is taken. Since the probe 30 is controled by dentists to contact with a tooth, it is inevitable that contact pressure varies with time, however, taking an image at the timing of a predetermined contact pressure enables an image of reflected light to be always taken at a constant contact pressure. The predetermined contact pressure can be appropriately determined depending on conditions of a tooth that is to be measured, and preferably 3.0 N or less, more preferably 2.5 N or less, even more preferably 2.0 N or less, and preferably 0.1 N or more, more preferably 0.5 N or more, even more preferably 0.8 N or more.

Although not shown in the figures, the device may be further equipped with a switch in addition to the pressure sensor 61, and the light receiving element 40 may take an image in response to a signal from the switch. Such a configuration enables an image to be taken at any time even when the end portion 31 of the probe 30 is non-contact and the pressure sensor 61 shows no signal.

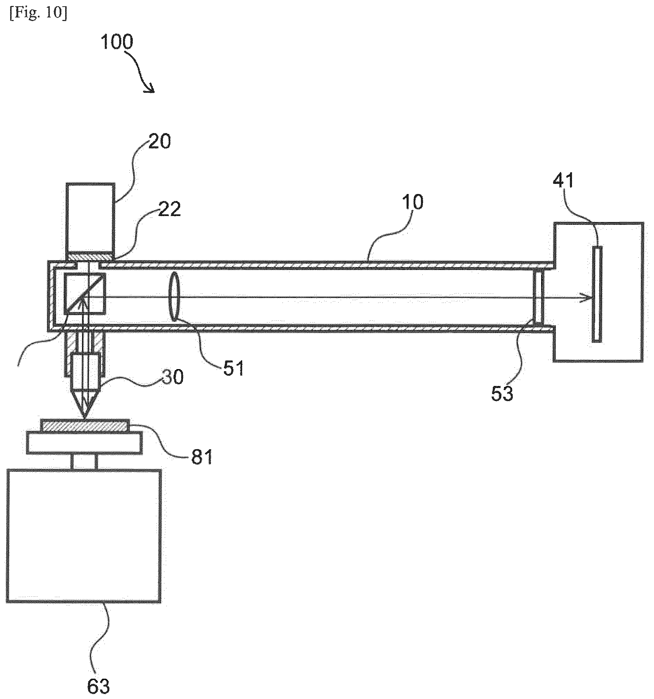

As shown in Fig. 10, the optical dental caries diagnostic device 1 of the present invention may further have a filter 53. By setting a wavelength of the light that can be transmitted through the filter 53 (transparent wavelength) the wave length of the light which the light source 20 emits, influence of light other than the light emitted by the light source 20 such as fluorescent lamp in the room and dental lighting equipment can be eliminated, which leads to a high S/N ratio.

The optical dental caries diagnostic device 1 of the present invention may further have a configuration where the device is housed in the housing 10. For example, an optical path from the light source 20 to the end portion 31 of the probe 30 (optical path e), an optical path from the end portion 31 of the probe 30 to the beam splitter 52 (optical path f), and an optical path from the beam splitter 52 to the light receiving element 40 (optical path g) may be placed in the housing 10, and the probe 30 may be attached to an opening of the housing 10.

The probe 30 is preferably attachable to and detachable from the opening of the housing 10. Thanks to such a configuration, the probe 30 can be sanitized or sterilized before and after measurement, or it can be replaced by another probe for another patient, or the best probe can be selected depending on conditions of a tooth to be measured. Furthermore, as consumables, it can be replaced by new one periodically, and it may be disposable for use for only one patient. During the replacement, the length of the optical path e, the optical path f, and the optical path g is preferably constant in each measurement. Accordingly, the housing 10 preferably has a fixing part such as a stopper for fixing the probe 30 in the opening to which or from which the probe 30 is attached or detached. Thanks to the constant length of the optical path a, the optical path b, and the optical path c, the replacement of the probe 30 has no influence on the imaging relationship, and stable imaging becomes available.

As shown in Fig. 2 and Fig. 3, a part of the housing 10 may be attached to a handle 70 having a cavity in which the housing 10 is contained. Fig. 2B is a cross-sectional view at another cross section of the optical dental caries diagnostic device 1 shown in Fig. 2A, and Fig. 3 is a cross-sectional view on line III of the optical dental caries diagnostic device 1 shown in Fig. 2B. In an embodiment, as shown in Fig. 2B and Fig. 3, the housing 10 can be attached to the handle 70 in a way where a rotation axis 62 disposed on the outside of the housing 10 is fitted on a shaft hole disposed in the handle 70. When the end portion 31 of the probe 30 is in contact with a tooth, the housing 10 revolves around the rotation axis 62. Force generated at the time can be detected by the pressure sensor 61. If the rotation axis 62 is disposed at the center between the point of effort at the side of the probe 30 and the point of load at the side of the pressure sensor 61, force acting on the probe 30 and force acting on the pressure sensor 61 can be matched.

Although not shown in the figures, in another embodiment, the rotation axis 62 may be disposed at an end part of the housing 10, which is on the side opposite to the light receiving surface of the light receiving element 40, so that the point of effort at the side of the probe 30 and the point of load at the side of the pressure sensor 61 are on the same side of the rotation axis 62. Such a configuration where the rotation axis 62 is disposed at an end part of the housing 10 enables the rotation axis 62 to penetrate the handle 70 and the housing 10, which can lead to a simple structure.

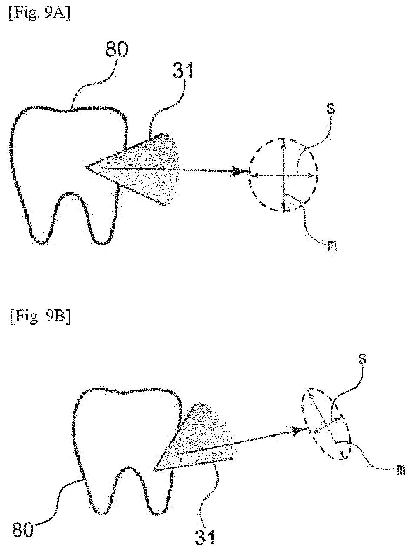

To obtain hardness quantitatively, an area of region showing weakened reflected light is preferably measured under a constant condition. For this reason, the optical dental caries diagnostic device of the present invention may have the following image processing process. The process will be described in reference to Fig. 8 and Fig. 9.

[Step of obtaining differential intensity image]

A differential intensity image (Fig. 8C) is obtained by subtracting a received light intensity by the light receiving element 40 while the end portion 31 of the probe 30 is in contact with a tooth (Fig. 8B) from a received light intensity by the light receiving element 40 while the end portion 31 of the probe 30 is in contact with nothing (Fig. 8A). Thanks to the subtraction, variation in quality of probes and received light caused by an object that is not the one to be measured can be eliminated. During the above process, the same probe should be used, and another imaging conditions should be the same.

[Step of binarizing differential intensity image]

The differential intensity image obtained by the above step is binarized in accordance with a predetermined threshold. In this process, the optical dental caries diagnostic device of the present invention may generate an error signal under the following conditions. While the probe 30 is preferably in contact with a surface of a tooth such that the central axis c of the probe 30 is as perpendicular to the surface of a tooth as possible as shown in Fig. 9A, it may not necessarily be perpendicular due to irregularities of the surface of a tooth and dentists' skill. If the angle between the central axis c and the surface of a tooth is not perpendicular, the shape of a region of weakened reflected light deviates from a circle as shown in Fig. 9B. Since it means that the obtained image includes information other than hardness, the device preferably generates an error signal when a ratio (a length of a major axis m / a length of a minor axis s) of a profile of the differential intensity image that is greater than the above threshold (the binarized differential intensity image) is greater than a predetermined value. The error signal generated when the ratio of the profile is greater than a predetermined value can eliminate information other than hardness.

[Step of obtaining area of region of binarized differential intensity image]

An area of the binarized differential intensity image processed in the above steps (Fig. 8D) is obtained. Since errors due to an angle of contact is eliminated, the area corresponds to hardness of a tooth, and therefore, the harder the tooth is, the smaller the area is; the softer the tooth is, the larger the area is.

The present inventions offers an optical diagnostic method for dental caries using the optical dental caries diagnostic device according to an embodiment of the present invention. The optical diagnostic method for dental caries includes preparing the optical dental caries diagnostic device according to an embodiment of the present invention, removing water from the end portion 31 of the probe 30, receiving light that is emitted by the light source 20 and reflected from the end portion 31 of the probe 30 to take an image, bringing the end portion 31 of the probe 30 into contact with a tooth, and receiving light that is emitted by the light source 20 and reflected from the end portion 31 of the probe 30 by the light receiving element 40 to take an image while the end portion 31 of the probe 30 is in contact with the tooth.

Since the oral cavity has an environment that is rich in water such as saliva and remained gargle water, such water may attach to the end portion of the probe. On top of water, adhering substance such as debris may remain, and such water and adhering substance need to be removed, because they may affect reflection of light from the end portion of the probe.

The present invention also provides an optical diagnostic device for periodontal diseases. Periodontal diseases are conventionally diagnosed by observing color of swollen gums or periodontal pocket depth, and the condition of gums cannot be examined quantitatively. Since gums become soft when they become inflamed to swell, progression of gum inflammation can be quantitatively diagnosed by using the optical diagnostic device for periodontal diseases of the present invention.

The optical diagnostic device for periodontal diseases of the present invention has the same fundamentals as the optical dental caries diagnostic device, and alternatively has a probe for gums. Appropriate selection of materials for the probe, the angle θ of the end portion of the probe, and the curvature radius of the R portion of the end portion of the probe enables hardness of gums to be precisely and quantitatively measured in such a way that the patient feels no pain. Furthermore, when a pressure sensor is used, the predetermined pressure at which the pressure sensor generates a signal of taking an image may be set so that it is suitable for gums.

The present invention further provides a diagnostic system for dental caries including the above described optical dental caries diagnostic device. The diagnostic system for dental caries has an information terminal device connected by wire or wirelessly to the optical dental caries diagnostic device, and optical dental caries diagnostic information (measured with the optical dental caries diagnostic device of the present invention) stored in the information terminal device. The diagnostic system for dental caries may also have dental caries diagnostic information measured by conventional methods for diagnosis of dental caries such as a dental probe, a cariotester, and panoramic radiograph. The system storing both diagnostic result by conventional methods and measured result by the optical dental caries diagnostic device of the present invention can compare the diagnostic result by conventional methods with the measured hardness obtained by the optical dental caries diagnostic device of the present invention. Consequently, clear diagnostic standard in dental caries diagnosis can be provided.

The diagnostic system for dental caries of the present invention can not only diagnose dental caries case by case, but also make the stored data shared among dentists as bigdata. The diagnostic system for dental caries according to an embodiment of the present invention may have software that offers advice based on the stored data when dentists make diagnoses and give treatment. In particular, the advice includes support to dentists to determine treatment strategy including judgement of necessity of drilling teeth, selection of drug for remineralization, and judgement of necessity of applying drug, which can eliminate variation in criterion for treatment among dentists. Furthermore, communication between information terminal devices enables telemedicine, which contributes to elimination of regional gap.

The present application claims priority based on Japanese Patent Application No. 2018-196773 filed on October 18, 2018. All the contents described in Japanese Patent Application No. 2018-196773 filed on October 18, 2018 are incorporated herein by reference.

EXAMPLES

A simulator 100 of the optical dental caries diagnostic device (shown in Fig. 10) was constructed to measure the hardness of samples. The simulator 100 had the following components.

   Light source: LED (center wavelength: about 635nm, M625L3 manufactured by Thorlabs Japan Inc.)
   Probe: made of glass (#49-397 manufactured by Edmund Optics)
   Light receiving element: black-and-white CMOS camera (#89-735 manufactured by Edmund Optics)
   Lens: AC127-025-A-ML (focal length: 25 mm, manufactured by Thorlabs Japan Inc.)
   Beam splitter: CCM5-BS016/M (transmittance: 50%, reflectivity: 50%, manufactured by Thorlabs Japan Inc.)
   Iris: SM1D12C (manufactured by Thorlabs Japan Inc.)
   Filter: FL635-10 (transparent wavelength: 630-640 nm, manufactured by Thorlabs Japan Inc.)
   Force gauge: ZTS-20N (manufactured by Imada Co., Ltd.)

Since the glass probe #49-397 manufactured by Edmund Optics has a surface coated with aluminum, the aluminum was removed by soaking the probe in hydrochloric acid, and was used for the measurement.

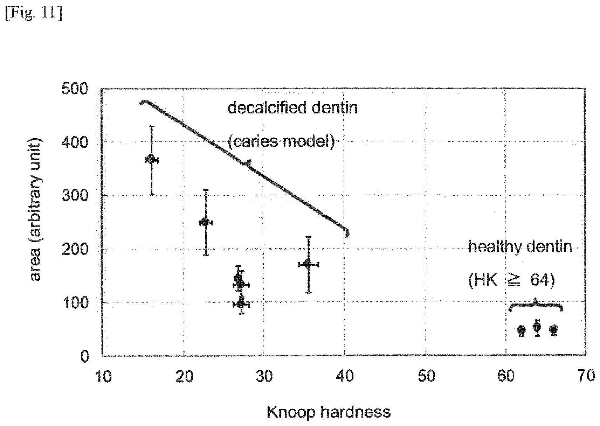

The probe of the simulator 100 was brought into contact with samples of bovine dentin, and the intensity of reflected light from the end portion of the probe was taken as an image when the force gauge showed 1.5 N (nearly equal to 150 gf). The measurement was repeated at 5 different points randomly selected on one sample. At the same time, Knoop hardness of the samples were measured. The results are shown in Fig. 11.

The results showed that the lower the Knoop hardness, the larger the measured area, which means that the hardness of the samples can be measured by the simulator 100. In addition, the samples of decalcified dentin (dental caries model) not only showed larger area than that of the samples of healthy dentin, the Knoop hardness of which was 64 or more, but also the relationship between the Knoop hardness and the measured area was found depending on progression of decalcification. That is, the result of the samples of decalcified dentin showed that the lower the Knoop hardness, the larger the measured area. These results shows that discrimination of a tooth that needs to be drilled from a tooth that needs preventive care was available.

DESCRIPTION OF REFERENCE SIGNS

   1: optical dental caries diagnostic device

10: housing
20: light source
22: iris
30: probe
31: end portion of probe 30
32: tip of probe 30
40: light receiving element
41: black-and-white CMOS camera
51: lens
52: beam splitter
53: filter
61: pressure sensor
62: rotation axis
63: force gauge
70: handle
80: tooth
81: sample of dentin
100: simulator of the optical dental caries diagnostic device

**Claims**

1. An optical dental caries diagnostic device, comprising:

   a probe having optical transparency and a taper shape with an end portion to be in contact with a tooth,
   a light source emitting light to the probe, and
   a light receiving element having a light receiving surface and receiving light from the end portion of the probe.

2. The optical dental caries diagnostic device according to claim 1, further comprising a lens disposed at a position where the end portion of the probe and the light receiving surface of the light receiving element establish an imaging relationship.

3. The optical dental caries diagnostic device according to claim 1 or 2, wherein the light receiving element includes unit cells arrayed in two dimensions.

4. The optical dental caries diagnostic device according to any one of claims 1 to 3, further comprising a beam splitter disposed at a position that is in an optical path between the probe and the light source and is in an optical path between the probe and the light receiving element.

5. The optical dental caries diagnostic device according to any one of claims 1 to 4, further comprising a pressure sensor, wherein the light receiving element takes an image when the pressure sensor shows a predetermined pressure generated by the probe being in contact with a tooth.

6. The optical dental caries diagnostic device according to any one of claims 1 to 5, wherein a differential intensity image is obtained by subtracting a received light intensity by the light receiving element while the end portion of the probe is in contact with a tooth from a received light intensity by the light receiving element while the end portion of the probe is in contact with nothing.

7. The optical dental caries diagnostic device according to claim 6, wherein the differential intensity image is binarized in accordance with a predetermined threshold to obtain a binarized differential intensity image.

8. The optical dental caries diagnostic device according to claim 7, wherein the device generates an error signal when a ratio (a length of a major axis/a length of a minor axis) of a profile of the binarized differential intensity image is greater than a predetermined value.

9. The optical dental caries diagnostic device according to claim 7 or 8, obtaining an area of a region of the binarized differential intensity image over the threshold.

10. The optical dental caries diagnostic device according to any one of claims 1 to 9, further comprising a housing, wherein the probe is attachable to and detachable from the housing.

11. An optical diagnostic method for dental caries, comprising:

preparing the optical dental caries diagnostic device according to any one of claims 1 to 10;
removing water from the end portion of the probe;
receiving light that is emitted by the light source and reflected from the end portion of the probe by the light receiving element to take an image;
bringing the end portion of the probe into contact with a tooth; and
receiving light that is emitted by the light source and reflected from the end portion of the probe by the light receiving element to take an image while the end portion of the probe is in contact with the tooth.

12. A diagnostic system for dental caries including the optical dental caries diagnostic device according to any one of claims 1 to 10, comprising:

an information terminal device connected by wire or wirelessly to the optical dental caries diagnostic device; and
optical dental caries diagnostic information (measured with the optical dental caries diagnostic device according to any one of claims 1 to 10) stored in the information terminal device.

13. The diagnostic system for dental caries according to claim 12, wherein the information terminal device has dental caries diagnostic information measured with a device other than the optical dental caries diagnostic device according to any one of claims 1 to 10.

14. An optical diagnostic device for periodontal diseases, comprising:

a probe having optical transparency and a taper shape with an end portion to be in contact with a gum;
a light source emitting light to the probe, and
a light receiving element receiving light from the end portion of the probe.

[Fig. 1A]

[Fig. 1B]

[Fig. 1C]

[Fig. 2A]

[Fig. 2B]

[Fig. 3]

[Fig. 4]

[Fig. 5A]

[Fig. 5B]

[Fig. 6]

[Fig. 7]

[Fig. 8A]

[Fig. 8B]

[Fig. 8C]

[Fig. 8D]

[Fig. 9A]

[Fig. 9B]

[Fig. 10]

[Fig. 11]

## INTERNATIONAL SEARCH REPORT

| | International application No. |
|---|---|
| | PCT/JP2019/039804 |

A. CLASSIFICATION OF SUBJECT MATTER
Int.Cl. A61C19/04(2006.01)i, A61B10/00(2006.01)i, A61C19/06(2006.01)i

According to International Patent Classification (IPC) or to both national classification and IPC

B. FIELDS SEARCHED

Minimum documentation searched (classification system followed by classification symbols)
Int.Cl. A61C19/04, A61B10/00, A61C19/06

Documentation searched other than minimum documentation to the extent that such documents are included in the fields searched
| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2001-170088 A (NONOMURA, Tomosuke) 26 June 2001, claim 3, paragraphs [0014], [0017], [0018], | 1, 3, 10, 12-14 |
| Y | [0022]-[0036], fig. 4, 5 | 4 |
| A | (Family: none) | 2, 5-9, 11 |
| | | |
| Y | JP 2001-299699 A (KALTENBACH & VOIGT GMBH & CO.) 30 October 2001, claim 1 & US 2001/0023057 A1, claim 1 | 4 |

☐ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 05.11.2019 | 19.11.2019 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**REFERENCES CITED IN THE DESCRIPTION**

**Patent documents cited in the description**

- JP 2008029412 A
- JP 2010252911 A
- JP 2011112629 A
- JP 2018196773 A